# EUROPEAN PATENT APPLICATION

(11) **EP 3 323 428 A1**
(43) Date of publication of application: **23.05.2018**
(21) Application number: 16306500.6
(22) Date of filing: 17.11.2016
(51) Int. Cl.: A61K 45/06, A61K 31/47, A61P 35/00, A61P 35/02

(54) **SELECTIVE C-FLIP INHIBITORS AS ANTICANCER AGENTS**

(71) Applicant: CNRS Centre National de la Recherche Scientifique, 75016 Paris (FR); Université de Rennes 1, 35000 Rennes (FR); Université d'Orléans, 45100 Orléans (FR)
(72) Inventor: YAACOUB, Katherine, 35000 RENNES (FR); GUILLAUDEUX, Thierry, 35000 RENNES (FR); DANIELLOU, Richard, 45370 CLERY SAINT ANDRE (FR); LAFITE, Pierre, 45560 SAINT DENIS EN VAL (FR); ACI-SECHE, Samia, 45160 OLIVET (FR); BONNET, Pascal, 45160 OLIVET (FR)
(74) Representative: Cabinet Plasseraud

(57) **Abstract**

The present invention provides nine c-FLIP inhibitors that are useful in the treatment of cancer, alone or in combination with other chemotherapeutic agents, in particular with TRAIL-based chemotherapeutic agents. The present invention also relates to pharmaceutical compositions and kits comprising at least one of the nine c-FLIP inhibitors and their use in methods for the treatment of cancer, in particular to overcome chemoresistance or to improve sensitivity of tumors to TRAIL-based therapy.

## Description

### Background of the Invention

Cancer chemotherapy has gradually improved with the development of novel antitumor drugs. However, while treatment of certain malignancies with chemotherapy has been successful and encouraging, the effectiveness has often been limited by drug resistance of tumors. Indeed, resistance to chemotherapy remains a major obstacle in the treatment of human malignancies. In fact, many tumors (e.g., colon cancer, pancreatic cancer, glioblastoma, and prostate cancer) are intrinsically resistant to many of the more potent cytotoxic agents used in cancer therapy. Other tumors (e.g., breast cancer), initially sensitive, recur and are resistant to the initial therapeutic agents, and, very often, also resistant to other cancer drugs which were not used in the initial treatment. In addition to reducing clinical effectiveness, chemoresistance results in early termination of treatment, reduced relapse-free interval and survival.

Various mechanisms are attributed to chemoresistance, one of which is defects in apoptosis. Apoptosis (or programmed cell death) occurs normally during development and aging and as a homeostatic mechanism to maintain cell populations in tissues. Apoptosis also plays a fundamental role as a defense mechanism such as in immune reactions or when cells are damaged by disease or noxious agents. Failure of the apoptotic process is an important component of many human cancers as the inability of cells to undergo physiologically programmed apoptotic cell death is an inherent characteristic of their malignant transformation. Defects in apoptotic signaling and redundant survival mechanisms in malignant cells also contribute to drug resistance in various cancer types. The mechanisms of apoptosis, which are complex and regulated at several levels, have been extensively investigated. The increased understanding of cell apoptosis mechanisms has helped identifying some molecular components of the apoptosis signaling pathways as relevant targets for cancer therapeutic intervention. One of these molecular components is c-FLIP, the cellular FADD-like interleukin-1beta-converting enzyme inhibitory protein (Safa and Pollock, Cancers, 2011, 3: 1639-1671; Safa, Exp. Oncol., 2012, 34: 176-184; Safa, J. Carcinog. Mutagen., 2013, Suppl 6, doi:10.4172/2157-2518.S6-003; Fulda, Expert. Opin. Ther. Targets, 2016, 17: 195-201; Shirley and Micheau, Cancer Lett., 2013, 332: 141-150).

The c-FLIP protein is a catalytically inactive caspase-8/-10 homologue. It is a master anti-apoptotic regulator and resistance factor that suppresses tumor necrosis factor-α (TNF-α)-, Fas ligand (Fas-L)- and TNF-related apoptosis-inducing ligand (TRAIL)-induced apoptosis, as well as apoptosis triggered by chemotherapy agents in malignant cells. The protein c-FLIP binds to FADD (Fas-Associated protein with Death Domain) and/or caspase-8 or -10 and TRAIL receptor 4 (DR4) and receptor 5 (DR5) in a ligand-dependent and -independent fashion and forms an apoptosis inhibitory complex (AIC). This interaction in turn prevents death-inducing signaling complex (DISC) formation and subsequent activation of the caspase cascade.

The strategies used so far to target c-FLIP include the use of molecules causing its degradation and/or a decrease in its expression. For example, small-interfering RNAs (siRNAs) that specifically knockdown the expression of c-FLIP_{L} (the long isoform of c-FLIP) in diverse human cancer cell lines were found to increase TRAIL-induced DISC recruitment and to boost the efficacy of chemotherapeutic agents, thereby enhancing effector caspase stimulation and apoptosis (Longley et al., Oncogene, 2006, 25: 838-848). Small molecules causing degradation of c-FLIP as well as decrease in mRNA and protein levels of c-FLIP_{L} and c-FLIP_{S} (the long and short isoforms of c-FLIP, respectively) have been developed (Hernandez et al., J. Gastrointest. Surg., 2001, 5: 56-65; Mawji et al., Cancer Res., 2007, 67: 8307-8315).

Much effort is focused on developing other c-FLIP-targeted cancer therapies. However, since c-FLIP has significant structural similarity to caspase-8, it is very difficult to target c-FLIP directly because small molecules capable of blocking the recruitment of c-FLIP to the DISC could simultaneously inhibit the recruitment of caspase-8 and thereby inhibit apoptosis. Therefore, it is recognized in the art (see for example, Safa, Exp. Oncol., 2012, 34: 176-184) that there is a need to develop small molecules which target c-FLIP without inhibiting caspase-8.

### Summary of the Invention

The present invention provides several small molecules that are selective inhibitors of the anti-apoptotic protein c-FLIP. To the Inventors' best knowledge, these small molecules are the first non-peptidic small molecules to selectively inhibit c-FLIP by directly interacting with, and more specifically by directly binding to, the c-FLIP protein itself. As described in the Examples section below, the present Inventors have identified these molecules by using an *in silico* method and a library containing 1,880 compounds available from the National Cancer Institute (NCI) database. A homology 3D model of c-FLIP allowed the identification of nine small molecules exhibiting both the ability to bind to c-FLIP with high affinity and the highest selectivity for c-FLIP *versus* procaspase-8. *In vitro* assays using a human lung cancer cell line overexpressing c-FLIP confirmed the inhibitory effects of these 9 small molecules and their capacity to restore the caspase-8 apoptotic cascade.

Accordingly, in one aspect, the present invention relates to a selective c-FLIP inhibitor for use in the treatment of cancer, wherein said selective c-FLIP inhibitor is a small molecule having one of the following formulas: 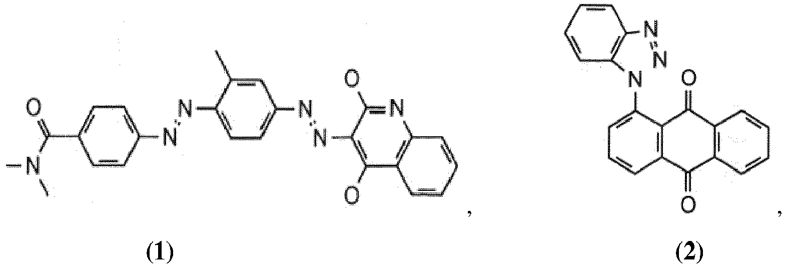 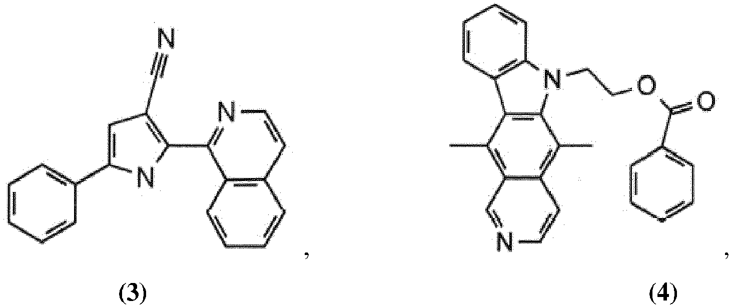 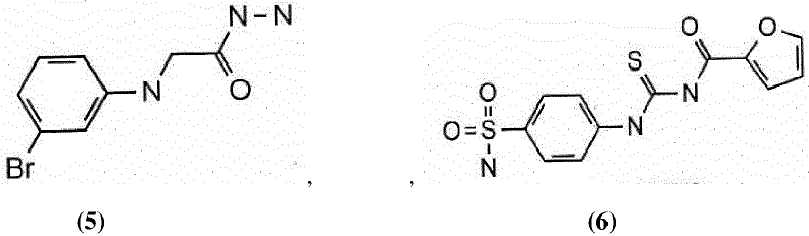 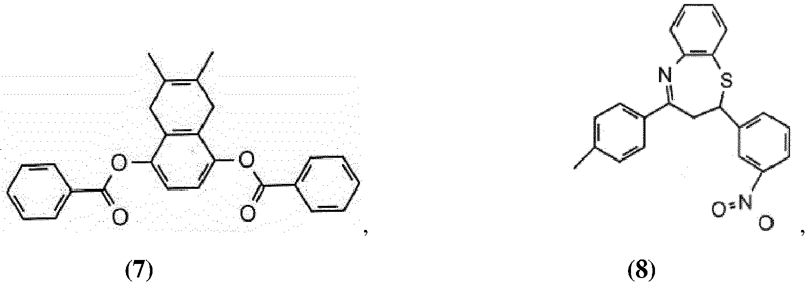 and 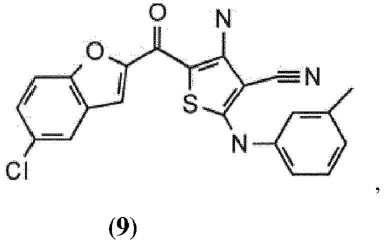 or a physiologically tolerable salt thereof.

In particular, the present invention provides the selective c-FLIP inhibitors, or physiological tolerable salts thereof, for their use as pro-apoptotic agents in the treatment of cancer.

In certain preferred embodiments, the selective c-FLIP inhibitor has one of formulas **(1), (4)** and **(9).**

In preferred embodiments, the subject is a cancer patient.

The selective c-FLIP inhibitors of the present invention may be used in the treatment of any cancer. However, in certain preferred embodiments, the cancer to be treated using one of the 9 selective c-FLIP inhibitors of the present invention is a c-FLIP-overexpressing cancer. For example, the c-FLIP-overexpressing cancer may be lung cancer, colorectal cancer, pancreatic cancer, ovarian cancer, gastric cancer, breast cancer, prostate cancer, melanoma, glioblastoma, bladder urothelial cancer, cervical cancer, Burkitt's lymphoma, non-Hodgkin's lymphoma, head and neck squamous cell carcinoma (HNSCC), hepatocellular carcinomas, B cell chronic lymphocytic leukemia, gallbladder carcinoma, Ewing sarcoma, nasopharyngeal carcinoma, follicular lymphoma, Acute Lymphoblastic Leukemia (ALL), neuroblastoma, myeloma, acute myeloid leukemia, osteosarcoma, renal sarcoma, endometrial carcinoma, malignant pleural mesothelioma, Kaposi's sarcoma (associated with the Kaposi's sarcoma herpes virus, which expresses the viral form of FLIP), peripheral T-cell lymphoma, and erythroleukemia.

In certain embodiments, the cancer to be treated using one of the 9 selective c-FLIP inhibitors of the present invention is chemoresistant. Thus, the present invention relates to a selective c-FLIP inhibitor, or a physiological tolerable salt thereof, for use in the treatment of a chemoresistant cancer. The present invention also relates to a selective c-FLIP inhibitor, or a physiological tolerable salt thereof, for use to overcome the chemoresistance of a cancer.

In certain embodiments, the cancer to be treated using one of the 9 selective c-FLIP inhibitors of the present invention is resistant or insensitive to a TRAIL-receptor agonist therapy (or to a TRAIL-based anticancer agent). Thus, the present invention also relates to a selective c-FLIP inhibitor, or a physiological tolerable salt thereof, for use in the treatment of a cancer that is resistant or insensitive to a TRAIL-receptor agonist therapy (or to a TRAIL-based anticancer agent). The present invention further relates to a selective c-FLIP inhibitor, or a physiological tolerable salt thereof, for use to overcome resistance or insensitivity of a cancer to a TRAIL-receptor agonist therapy (or to a TRAIL-based anticancer agent) or to improve the sensitivity of a cancer to a TRAIL-receptor agonist therapy (or to a TRAIL-based anticancer agent).

In certain embodiments, the TRAIL-based anticancer agent is a TRAIL receptor agonist or ligand. In particular, the TRAIL receptor agonist or ligand may be a TRAIL-R1 agonist or ligand or a TRAIL-R2 agonist or ligand.

In a related aspect, the present invention provides a method for treating cancer in a subject, the method comprising a step of administering to the subject in need thereof a therapeutically effective amount of one of the 9 selective c-FLIP inhibitors, or a physiologically acceptable salt thereof, or of a pharmaceutical composition thereof. As indicated above, in certain embodiments, the cancer overexpresses c-FLIP. In certain embodiments, the cancer is a chemoresistant cancer. In certain embodiments, the cancer is resistant or insensitive to a TRAIL-receptor agonist therapy (or to a TRAIL-based anticancer agent).

In another aspect, the present invention provides a pharmaceutical composition for use in the treatment of cancer or for use to overcome the chemoresistance of a cancer or for use to overcome the resistance of a cancer to a TRAIL-receptor agonist therapy (or to a TRAIL-based anticancer agent) or to improve the sensitivity of a cancer to a TRAIL-receptor agonist therapy (or to a TRAIL-based anticancer agent), wherein said pharmaceutical composition comprises an effective amount of at least one selective c-FLIP inhibitor having one of formulas **(1)-(9)** and a pharmaceutically acceptable carrier or excipient thereof.

In certain embodiments, the pharmaceutical composition comprises at least one additional therapeutic agent. For example, the additional therapeutic agent may be selected from the group consisting of anti-cancer agents, anti-inflammatory agents, immunomodulatory agents, analgesics, antimicrobial agents, antibacterial agents, antibiotics, antioxidants, antiseptic agents, and combinations thereof.

In certain embodiments, the additional therapeutic agent is a TRAIL-based anticancer agent, preferably a TRAIL receptor agonist or ligand, more preferably a TRAIL-R1 agonist or ligand or a TRAIL-R2 agonist or ligand.

In yet another aspect, the present invention provides a kit for use in the treatment of cancer or for use to overcome the chemoresistance of a cancer, wherein said kit comprises at least one selective c-FLIP inhibitor having one of formulas **(1)-(9),** or a pharmaceutical composition thereof, and at least one additional therapeutic agent, wherein the at least one selective c-FLIP inhibitor and at least one additional therapeutic agent are comprised in separate containers.

In particular, the kit may be provided for use to overcome the resistance of a cancer to a TRAIL-receptor agonist therapy (or to a TRAIL-based anticancer agent) or to improve the sensitivity of a cancer to a TRAIL-receptor agonist therapy (or to a TRAIL-based anticancer agent), wherein said kit comprises at least one selective c-FLIP inhibitor having one of formulas **(1)-(9),** or a pharmaceutical composition thereof, and at least one TRAIL-based anticancer agent, wherein the at least one selective c-FLIP inhibitor and at least one TRAIL-based anticancer agent are comprised in separate containers. The TRAIL-based anticancer agent is preferably a TRAIL receptor agonist or ligand, and more preferably a TRAIL-R1 agonist or ligand or a TRAIL-R2 agonist or ligand.

In a related aspect, the present invention relates to the use of at least one of the nine selective c-FLIP inhibitors as described herein for the manufacture of a medicament, in particular of a medicament intended to be used in an anti-cancer therapy.

These and other objects, advantages and features of the present invention will become apparent to those of ordinary skill in the art having read the following detailed description of the preferred embodiments.

### Brief Description of the Drawing

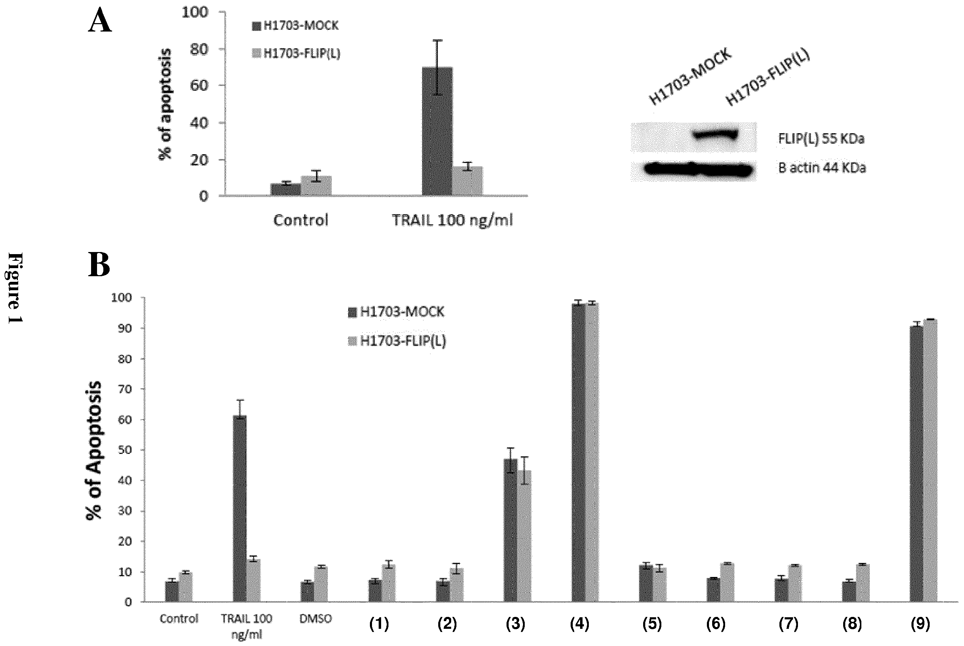

**Figure 1****. Sensitivity of cells of the H1703 cell line to TRAIL and c-FLIP inhibitors described herein.** Mock cells were transfected with an empty vector, while H17003-LFIP(L) expressed the long form of the c-FLIP protein. (A) H1703 cells were tested using the death ligand TRAIL at 100 ng/mL for 18 hours. The level of apoptosis reached more than 60% in H1706-Mock. In contrast, the overexpression of c-FLIP (L) was found to inhibit TRAIL-mediated apoptosis. **(B)** The 9 newly identified c-FLIP inhibitors **((1)** to **(9))** were tested first at a standard concentration of 25 µM. Control was performed using DMSO alone. The molecules that were found to be highly toxic at that concentration, were later used at a lower concentration; while the molecules that were found to exhibit no toxicity at that concentration, were later used at 25 µM or higher concentrations.

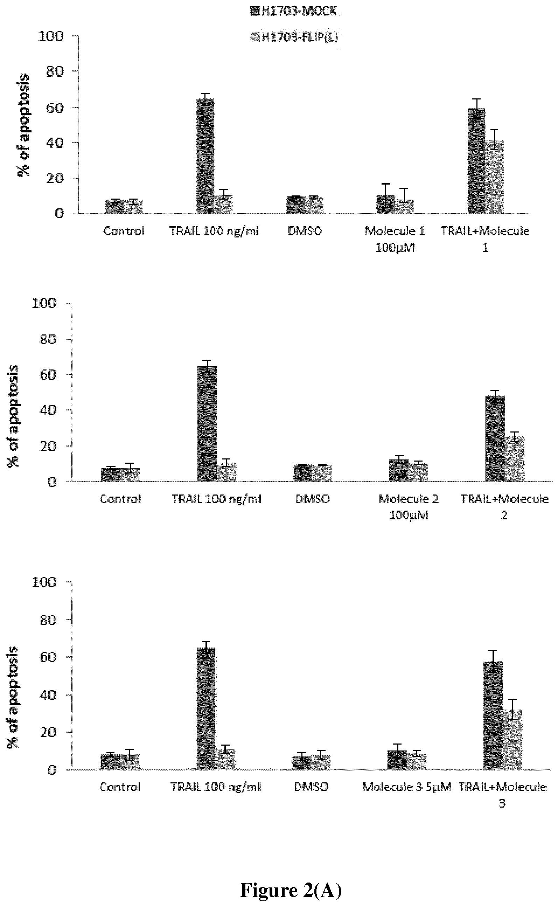

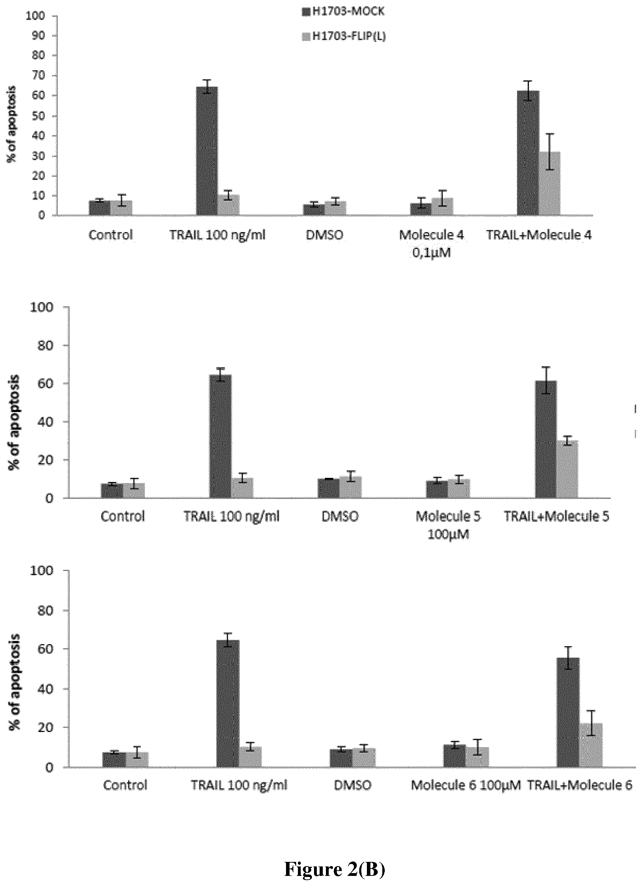

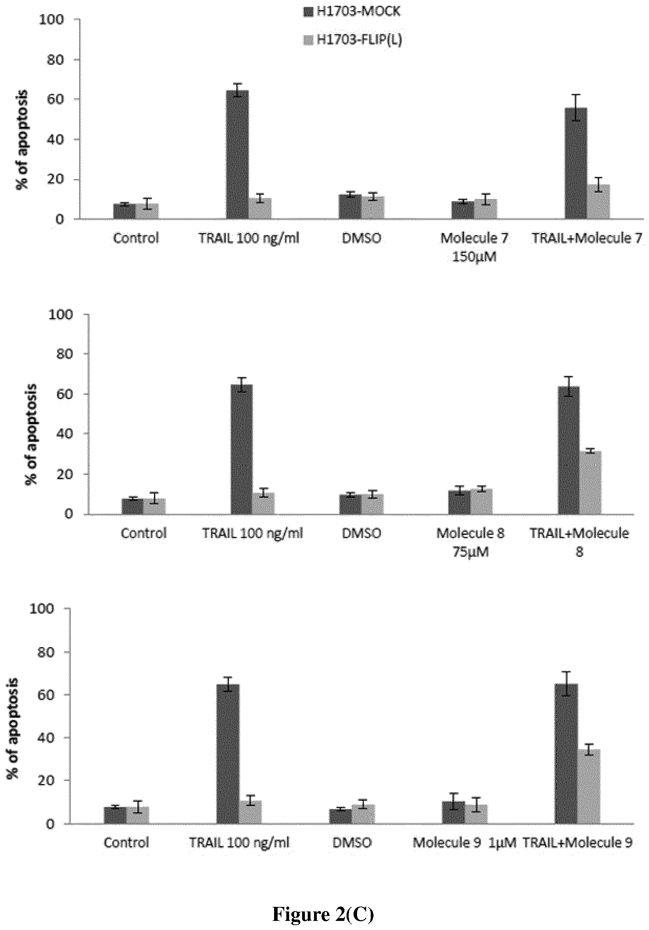

**Figure 2****. The c-FLIP inhibitors sensitize H1706 cells to apoptotic cell death when combined with the death ligand TRAIL.** To determine the appropriate concentration for each molecule, a range of concentrations was used, from 25 µM and up for the non-toxic compounds and from 25 µM and down for the toxic compounds. After determination of the most suitable concentration, each compound was tested alone or in combination with TRAIL to determine if this co-administration can restore apoptosis in cancer cell. **(A)** Results obtained for molecules **(1), (2)** and **(3). (B)** Results obtained for molecules **(4), (5)** and **(6). (C)** Results obtained for molecules **(7), (8)** and **(9).** The most active compounds were found to be molecules **(1), (3), (4), (8)** and **(9).**

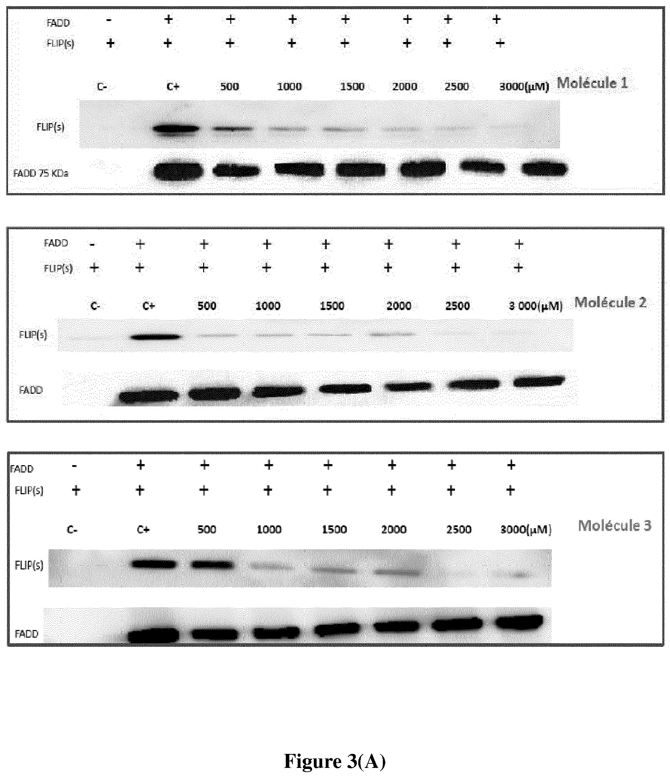

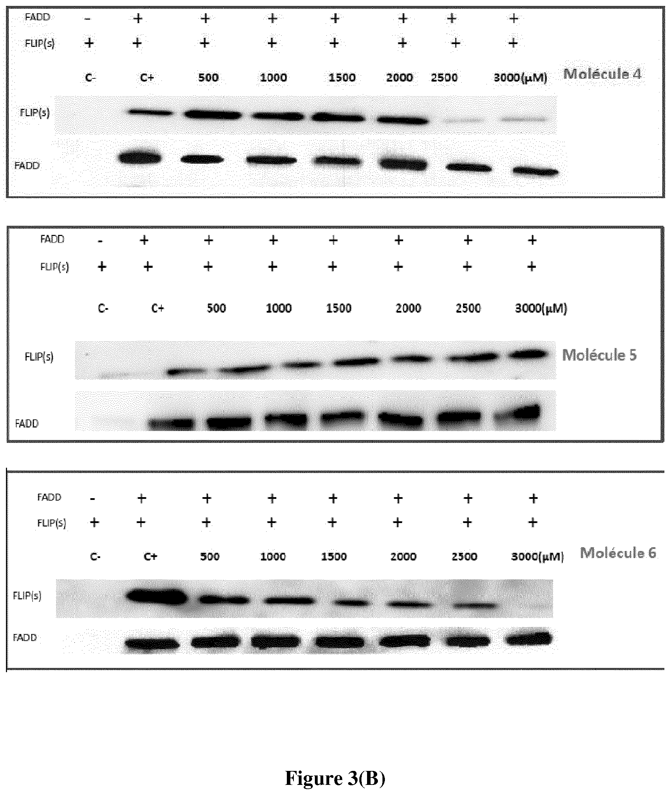

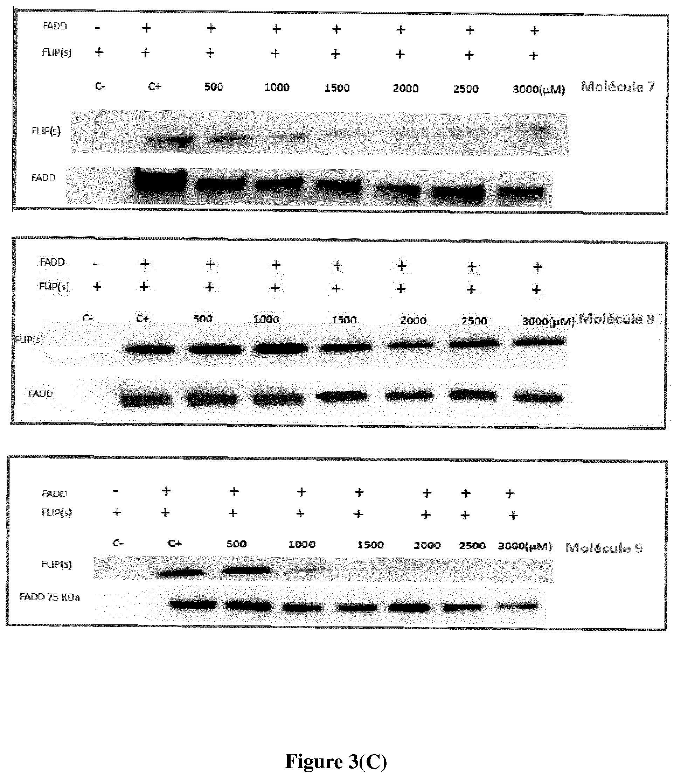

**Figure 3****. Inhibition of cFLIP/FADD interaction by the c-FLIP inhibitors.** Pull down assays were performed to assess the inhibitory activity of the 9 c-FLIP inhibitors on the prevention of FLIP/FADD interaction. The FLIP and FADD molecules were produced and purified on chromatography columns, and then incubated in the presence of each molecule at different concentrations. The samples were later passed on MBP-columns to FADD. The inhibitory activity of the different molecules was assessed by **(A)** Results obtained for molecules **(1), (2)** and **(3). (B)** Results obtained for molecules **(4), (5)** and **(6). (C)** Results obtained for molecules **(7), (8)** and **(9).**

### Definitions

Throughout the specification, several terms are employed that are defined in the following paragraphs.

As used herein, the term *"subject"* refers to a human or another mammal (e.g., primate, dog, cat, goat, horse, pig, mouse, rat, rabbit, and the like), that can develop cancer, but may or may not be suffering from the disease. Non-human subjects may be transgenic or otherwise modified animals. In many embodiments of the present invention, the subject is a human being. In such embodiments, the subject is often referred to as an ***"individual"*** or a ***"patient".*** These terms do not denote a particular age, and thus encompass newborns, children, teenagers, and adults. The term "patient" more specifically refers to an individual suffering from the disease. Thus, the term ***"cancer patient"*** refers to an individual suffering from a cancer. A cancer patient may or may not have been diagnosed with cancer. The term also includes individuals that have previously undergone therapy for cancer.

As used herein, the term ***"cancer"*** refers to or describes the physiological condition in mammals that is typically characterized by unregulated cell growth, lack of differentiation and ability to invade local tissues and metastasize. Cancer can develop in any tissue of any organ. Examples of cancers include, but are not limited to carcinoma, lymphoma, blastoma, sarcoma, and leukemia. More particularly, examples of such cancers include bone cancer, lung cancer, liver cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, uterine cancer, carcinoma of the sexual and reproductive organs, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the bladder, cancer of the kidney, renal cell carcinoma, carcinoma of the pelvis, neoplasms of the central nervous system (CNS), neuroectodermal cancer, spinal axis tumors, glioma, meningioma, and pituitary adenoma.

The terms ***"aggressive"*** and ***"invasive"*** are used herein interchangeably. When used herein to characterize a cancer, they refer to the proclivity of a tumor for expanding beyond its boundaries into adjacent tissue. Invasive cancer can be contrasted with organ-confined cancer wherein the tumor is confined to a particular organ. The invasive property of a tumor is often accompanied by the elaboration of proteolytic enzymes, such as collagenases, that degrade matrix material and basement membrane material to enable the tumor to expand beyond the confines of the capsule, and beyond confines of the particular tissue in which that tumor is located.

The term ***"metastasis",*** as used herein, refers to the spread of tumor cells from one organ or tissue to another location. The term also refers to tumor tissue that forms in a new location as a result of metastasis. A "metastatic cancer" is a cancer that spreads from its original, or primary, location, and may also be referred to as a "secondary cancer" or "secondary tumor". Generally, metastatic tumors are named for the tissue of the primary tumor from which they originate. The process of tumor metastasis is a multistage event involving local invasion and destruction of intercellular matrix, intravasation into blood vessels, lymphatics or other channels of transport, survival in the circulation, extravasation out of the vessels in the secondary site and growth in the new location.

The term ***"chemoresistant cancer"*** refers to a cancer that exhibits chemotherapy resistance or chemoresistance. The terms ***"chemotherapy resistance"*** and ***"chemoresistance"*** are used herein interchangeably. They refer to the insensitivity of cancer cells to chemotherapy, *i.e.* to the ability of cancer cells to avoid the intended therapeutic cytotoxic effects of a chemotherapeutic agent. Chemoresistance can be intrinsic or can develop over time, for example after repeated exposures to anticancer therapy. In contrast, ***"chemosensititivy"*** refers to the susceptibility of cancer cells to the cytotoxic effects of a chemotherapeutic agent. Changes in chemosensitity and chemoresistance can be measured by comparing the toxic effects of chemotherapy after a single treatment (*e.g*., in different patients) or by assessing changes in toxicity over the course of repeated treatments (*e.g*., in the same patient or different patients). An increase in chemosensitivity or decrease in chemoresistance of a cancer cell means an improvement in the therapeutic efficacy of a chemotherapeutic agent.

The term ***"treatment,"*** is used herein to characterize a method or process that is aimed at (1) delaying or preventing the onset of a disease or condition; (2) slowing down or stopping the progression, aggravation, or deterioration of at least one symptom of the disease or condition; (3) bringing about amelioration of at least one symptom of the disease or condition; or (4) curing the disease or condition. A treatment may be administered after initiation of the disease or condition, for a therapeutic action. Alternatively, a treatment may be administered prior to the onset of the disease or condition, for a prophylactic or preventive action. In this case, the term ***prevention"*** is used.

The term ***"combination therapy"*** refers to a treatment of a disease or symptom thereof, or to a method for achieving a desired physiological change, which includes administering an effective amount of two or more chemical agents or components to treat a disease or symptom thereof, or to produce a physiological change, wherein the chemical agents or components are administered together, such as part of the same composition, or administered separately and independently at the same time or at different times (*i.e.,* administration of each agent or component is separated by a finite period of time from each other). The term ***"combination therapy"*** also refers to a treatment of a disease or symptom thereof, or to a method for achieving a desired physiological change, including administering an effective amount of at least one chemical agent or compound and at least one therapeutic procedure *(e.g.,* surgery, radiotherapy, etc...), wherein the chemical agent or compound and the therapeutic procedure are administered together or separately and independently.

As used herein, the term ***"dosage regimen"*** refers to the schedule of drug administration, including formulation, route of administration, drug dose, dosing interval and treatment duration.

The term ***"pro-apoptotic",*** used herein to define or characterize a molecule, compound, agent or composition, refers to a molecule, compound, agent or composition that induces, causes, promotes, or increases apoptosis or programmed cell death, irrespective of its mechanism of action.

A ***"pharmaceutical compositions"*** is defined herein as comprising an effective amount of at least one selective c-FLIP inhibitor of the invention or a physiologically tolerable salt thereof, and at least one pharmaceutically acceptable carrier or excipient.

The term ***"physiologically tolerable salt"*** refers to any acid addition or base addition salt that retains the biological activity and properties of the free base or free acid, respectively, and that is not biologically or otherwise undesirable. Acid addition salts are formed with inorganic acids (*e.g*., hydrochloric, hydrobromic, sulfuric, nitric, phosphoric acids, and the like); or organic acids (*e.g.,* acetic, propionic, pyruvic, maleic, malonic, succinic, fumaric, tartaric, citric, benzoic, mandelic, methanesulfonic, ethanesulfonic, *p*-toluenesulfonic, salicylic acids, and the like). Base addition salts can be formed with inorganic bases (*e.g*., sodium, potassium, lithium, ammonium, calcium, magnesium, zinc, aluminum salts, and the like) or organic bases (*e.g*., salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-dimethyl-aminoethanol, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperazine, piperidine, N-ethylpiperidine, polyamine resins, and the like).

As used herein, the term ***"therapeutically effective amount"*** refers to any amount of a molecule, compound, agent, or composition that is sufficient to fulfil its intended purpose(s), *e.g.,* a desired biological or medicinal response in a cell, tissue, system or subject.

The term ***"pharmaceutically acceptable carrier or excipients"*** refers to a carrier medium which does not interfere with the effectiveness of the biological and/or therapeutic activity of the active ingredient(s) and which is not excessively toxic to the host at the concentration at which it is administered. The term includes solvents, dispersion media, coatings, antibacterial and antifungal agents, isotonic agents, and adsorption delaying agents, and the like. The use of such media and agents for pharmaceutically active substances is well known in the art (see for example *"*Remington's Pharmaceutical Sciences", E.W. Martin, 18th Ed., 1990, Mack Publishing Co.: Easton, PA, which is incorporated herein by reference in its entirety).

The terms ***"approximately"*** and ***"about",*** as used herein in reference to a number, generally include numbers that fall within a range of 10% in either direction of the number (greater than or less than the number) unless otherwise stated or otherwise evident from the context (except where such number would exceed 100% of a possible value).

### Detailed Description of Certain Preferred Embodiments

As mentioned above, the present invention provides nine selective c-FLIP inhibitors that are useful as anti-cancer agents, in particular in the treatment of cancers overexpressing c-FLIP.

### I - Selective c-FLIP Inhibitors

As used herein, the term **"c-FLIP"** refers to a human protein called cellular FLICE-like inhibitory protein or cellular FADD-like interleukin-1beta-converting enzyme inhibitory protein (UniProtKB/Swiss-Prot Number: 015519). This protein is also known as: FLIP; CASH; Casper; i-FLICE; MRIT; FLAME-1; Usurpin; and CLARP. It is encoded by the *CLFAR* gene which is situated on the long (q) arm of human chromosome 2 at position 33.1 (Gene ID: 8837). c-FLIP has 13 distinct spliced variants, three of which are expressed as proteins: the 26 kDa short form (c-FLIP_{S}), the 24 kDa form of c-FLIP (c-FLIP_{R}) and the 55 kDa long form (c-FLIP_{L}) (Safa, Exp. Oncol., 2012, 34: 176-184).

The term **"c-FLIP *inhibitor***" refers to a compound or agent which prevents, inhibits, reduces, alleviates or decreases the anti-apoptotic activity of c-FLIP. The terms "prevent", "inhibit", "reduce", "alleviate" and "decrease" are used relative to a control. One skilled in the art would readily identify the appropriate control to use for each experiment. For example, an inhibited c-FLIP-anti-apoptotic activity in a subject or cell treated with a c-FLIP inhibitor is compared to a response in a subject or cell that is not treated with the c-FLIP inhibitor. In the context of the present invention, the nine selective c-FLIP inhibitors inhibit the anti-apoptotic activity of c-FLIP by directly interacting with c-FLIP, and more specifically by directly binding to c-FLIP.

As used herein, the term "***selective* c-FLIP *inhibitor***" refers to a c-FLIP inhibitor that prevents, inhibits, reduces, alleviates or decreases the anti-apoptotic activity of the protein c-FLIP by binding to c-FLIP in a preferential manner (*i.e.,* by binding to c-FLIP with higher affinity than to other proteins, in particular by binding to c-FLIP with a higher affinity than to caspase-8). Binding can be measured using a variety of methods standard in the art including enzyme immunoassays (*e.g*., enzyme linked immunoassays (ELISA)), immunoblot assays, and the like (see, Sambrook et al., Eds., Molecular Cloning: A Laboratory Manual, 2nd Ed., Cold Spring Harbor Laboratory Press, 1989, or Harlow and Lane, Eds., Using Antibodies, Cold Spring Harbor Laboratory Press, 1999).

The nine selective c-FLIP inhibitors identified by the present Inventors are small molecules having one of the formulas **(1)** to **(9).** The invention also encompasses any physiologically tolerable salt of these nine selective c-FLIP inhibitors.

The selective c-FLIP inhibitors according to the present invention may be prepared using any of a variety of suitable methods known in the art, since the method of preparation is not a limiting factor of the invention. Alternatively, the selective c-FLIP inhibitors according to the present invention may be purchased (Molecule **(1):** NCI Diversity 3; Molecule **(2):** NCI Diversity 3; Molecule **(3):** NCI diversity 3, NCI plated 2007; Molecule **(4):** NCI diversity 3, NCI plated 2007; Molecule **(5):** NCI diversity 3, NCI plated 2007, Vitas M, eMolecules, Molport BB; Molecule **(6):** NCI diversity 3, NCI plated 2007, vitas M, eMolecules, pharmeks, Asinex, Molport; Molecule **(7):** NCI diversity 3; Molecule **(8):** NCI diversity 3, eMolecules, Molport, life chemicals, Mcule Make-on-demand; Molecule **(9):** NCI diversity 3, NCI plated 2007).

### II - Therapeutic Uses of the Selective c-FLIP Inhibitors

### A. Indications

A selective c-FLIP inhibitor according to the present invention may be used in the treatment of cancer. In particular, a selective c-FLIP inhibitor according to the present invention may be used as a pro-apoptotic agent in the treatment of cancer. A method of treatment of the invention may be accomplished using a selective c-FLIP inhibitor (as defined herein), or a pharmaceutical composition thereof. The method generally comprises a step of administering to a subject in need thereof, an effective amount of a selective c-FLIP inhibitor of the invention, or a pharmaceutical composition thereof. Generally, the subject is a cancer patient.

In some embodiments, the selective c-FLIP inhibitors and methods of treatment of the present invention are used as a first-line therapy (sometimes called primary therapy). In other embodiments, the selective c-FLIP inhibitors and methods of the present invention are used as a second-line therapy or a third-line therapy. In still other embodiments, the selective c-FLIP inhibitors and methods of the present invention are used as a salvage therapy. The term ***"salvage therapy",*** as used herein, means a therapeutic agent that can be taken with any regimen after a subject's initial treatment regimen has failed or after the subject's condition has not responded to an initial treatment. In yet other embodiments, the selective c-FLIP inhibitors and methods of the present invention are used as a rescue therapy. The term ***"rescue therapy",*** as used herein, refers to a therapeutic agent that is used to counteract the action of an initial treatment or to overcome resistance to a standard or an initial treatment. In other embodiments, the selective c-FLIP inhibitors and methods of the present invention are used as a neoadjuvant therapy *(i.e.,* are administered to a subject before a main or first line treatment, for example to reduce the size or extent of the cancer to be treated before administration of the main or first treatment). In other embodiments, the selective c-FLIP inhibitors and methods of the present invention are used as an adjuvant therapy *(i.e.,* are administered to a subject to modify the effect of one or more other therapeutic agents that are already administered to the subject or are concurrently administered to the subject or subsequently administered to the subject).

In the practice of a method of treatment according to the present invention, the cancer to be treated with a selective c-FLIP inhibitor as described herein, may be any cancer developed in any tissue of any organ. Thus, the cancer may be a carcinoma, lymphoma, blastoma, sarcoma, and leukemia. Examples of cancers include, but are not limited to, bone cancer, lung cancer, liver cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, uterine cancer, carcinoma of the sexual and reproductive organs, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the bladder, cancer of the kidney, renal cell carcinoma, carcinoma of the pelvis, neoplasms of the central nervous system (CNS), neuroectodermal cancer, spinal axis tumors, glioma, meningioma, and pituitary adenoma.

In certain preferred embodiments, the cancer to be treated using a method of the present invention is known to overexpress c-FLIP. Increased expression of c-FLIP isoforms has been shown in cell lines from various types of cancers including, but not limited to, colorectal cancer (Longley et al., Oncogene, 2006, 25: 838-848; Wilson et al., Cancer Res., 2007, 6: 5754-5762), pancreatic cancer (Haag et al., Gut, 2011, 60: 225-237; Kauh et al., PLoS One, 2010, 5: e10376), ovarian cancer (El-Gazzar et al., Gynecol. Oncol., 2010, 117: 451-459; Park et al., Biochem. Pharmacol., 2009, 77: 1328-1336), gastric cancer (Nam et al., Cancer Sci., 2003, 94: 1066-1073), breast cancer (Day et al., Biochem. Pharmacol., 2008, 76:1694-1704; Tiwary et al., PLoS One, 2010, 5: e11865), prostate cancer (Zhang et al., Cancer Res., 2007, 67: 9425-9434), melanoma (Yang et al., Clin. Exp. Pharmacol. Physiol., 2007, 34, 1245-1251), and gliobastoma (McLornan et al., Clin. Cancer Res., 2010, 16: 3442-3451). Elevated levels of c-FLIP in tumor tissue from patients with colorectal cancer (McLornan et al., Clin. Cancer Res., 2010, 16: 3442-3451; Ullenhag et al., Clin. Cancer Res., 2007, 13: 5070-5075), bladder urothelial cancer (Korkolopoulou et al., Urology, 2004, 63: 1198-1204), cervical cancer (Wang et al., Gynecol. Oncol., 2007, 105: 571-577), Burkitt's lymphoma (Valnet-Rabier et al., Br. J. Haematol., 2005, 128: 767-773), non-Hodgkin's lymphoma (Valente et al., Br. J. Haematol., 2006, 132: 560-570), head and neck squamous cell carcinoma (HNSCC) (Li et al., J. Cancer Res. Clin. Oncol., 2008, 134: 609-615), lung adenocarcinomas (Salon et al., Cell Death Differ., 2006, 13: 260-272) and hepatocellular carcinomas (Du et al., Exp. Clin. Cancer Res., 2009, 28: 24) have been correlated with a poor clinical outcome. Overexpression of c-FLIP is also seen in gastric cancer and plays an important role in lymph node metastasis, which ultimately contributes to the tumor progression (Zhou et al., Clin. Sci. (Lond.), 2004, 106: 397-405). Other cancers associated with c-FLIP overexpression include B cell chronic lymphocytic leukemia, gallbladder carcinoma, Ewing sarcoma, nasopharyngeal carcinoma, follicular lymphoma, Acute Lymphoblastic Leukemia (ALL), neuroblastoma, myeloma, acute myeloid leukemia, osteosarcoma, renal sarcoma, endometrial carcinoma, malignant pleural mesothelioma, Kaposi's sarcoma, peripheral T-cell lymphoma, and erythroleukemia. A method of treatment according to the invention may therefore be used to treat any cancer associated with c-FLIP-overexpression.

It is worth noting that the c-FLIP inhibitors may be useful in certain anti-viral approaches, in particular in the cases of viruses expressing the viral form of FLIP such as in the treatment of Kaposi's sarcoma associated with the Kaposi's sarcoma herpes virus, which expresses the viral form of FLIP)

In certain embodiments, a method of treatment according to the present invention is used to overcome chemoresistance in a cancer patient, or in other words to treat a patient suffering from a chemoresistant cancer. Examples of pathological contexts in which a selective c-FLIP inhibitor of the invention, or a pharmaceutical composition thereof, can be beneficially administered include, but are not limited to, non-small cell lung cancer and forms of the disease having developed resistance to EGFR targeted therapies (Gefitinib, Erlotinib); hepatocellular carcinoma treated by Sorafenib and forms that have developed resistance to such agent; HER2-amplified breast cancer associated to HER2 targeted therapies (Trastuzumab) and forms that have developed resistance to this agent; Estrogen Receptor positive (ER+) breast cancer in association with hormone therapies and noticeably a subset of Estrogen Receptor positive (ER+) breast cancers with low levels of ER which do not respond to hormonal therapy (luminal B); some triple negative breast cancers, breast cancers developing resistance to Estrogen Receptor-targeted therapies (Fulvestant); gastrointestinal stromal tumors, being either insensitive or resistant to PDGFR targeted therapies (Imatinib); resistance to Androgen Receptor-targeted therapies in prostate cancer; resistance to BRAFV600E targeted therapies of melanoma and colon cancer; and the like. Other examples includes tamoxifen resistance in ER+ breast cancer, cetuximab resistance in head and neck cancers, trebananib resistance in renal and ovarian cancer, temozolomide resistance in glioblastoma, gemcitabine resistance in pancreatic cancer, carboplatin resistance in ovarian cancer, vincristine resistance in neuroblastoma, andmethotrexate resistance in acute lymphoblastic leukemia.

The effects of a treatment according to the present invention may be assessed or monitored using any of the diagnostic assays, tests and procedures known in the art.

### B. Combination Therapy

In certain embodiments, a selective c-FLIP inhibitor of the present invention, or a pharmaceutical composition thereof, is administered alone according to a method of treatment described herein. In other embodiments, a selective c-FLIP inhibitor of the present invention, or a pharmaceutical composition thereof, is administered in combination with at least one additional therapeutic agent or therapeutic procedure. The selective c-FLIP inhibitor, or pharmaceutical composition thereof, may be administered prior to administration of the therapeutic agent or therapeutic procedure, concurrently with the therapeutic agent or procedure and/or following administration of the therapeutic agent or procedure.

Therapeutic procedures that may be performed in combination with administration of a selective c-FLIP inhibitor of the invention, or a composition thereof, include, but are not limited to, surgery, radiotherapy, and the like.

Therapeutic agents that may be administered in combination with one of the selective c-FLIP inhibitors of the invention, or a pharmaceutical composition thereof, may be selected among a large variety of biologically active compounds that are known to have a beneficial effect in the treatment of cancer or that are known to be beneficial to a patient in general (*e.g.*, anti-cancer agents, anti-inflammatory agents, immunomodulatory agents, analgesics, antimicrobial agents, antibacterial agents, antibiotics, antioxidants, antiseptic agents, and combinations thereof).

Anti-cancer agents that may be administered in combination with a selective c-FLIP inhibitor described herein, or a pharmaceutical composition thereof, include drugs conventionally classified in one of the following groups: alkylating agents, purine antagonists, pyrimidine antagonists, plant alkaloids, intercalating antibiotics, aromatase inhibitors, anti-metabolites, mitotic inhibitors, growth factor inhibitors, cell cycle inhibitors, enzymes, topoisomerase inhibitors, biological response modifiers, anti-hormones and anti-androgens. Examples of such anti-cancer agents include, but are not limited to, BCNU, cisplatin, gemcitabine, hydroxyurea, paclitaxel, temozolomide, topotecan, fluorouracil, vincristine, vinblastine, procarbazine, decarbazine, altretamine, methotrexate, mercaptopurine, thioguanine, fludarabine phosphate, cladribine, pentostatin, cytarabine, azacitidine, etoposide, teniposide, irinotecan, docetaxel, doxorubicin, daunorubicin, dactinomycin, idarubicin, plicamycin, mitomycin, bleomycin, tamoxifen, flutamide, leuprolide, goserelin, aminogluthimide, anastrozole, amsacrine, asparaginase, mitoxantrone, mitotane and amifostine.

Other examples of such anti-cancer agents include therapeutic antibodies used in the treatment of cancer, including, but are not limited to, anti-CD52 antibodies such as alemtuzumab (CAMPATH™), which is used in the treatment of chronic lymphocytic leukemia; anti-VEGF antibodies including bevacizumab (AVASTIN™) which is used in the treatment of colorectal cancer and breast cancer; anti-CD33 antibodies, including gemtuzumab ozogamicin (MYLOTARG™) which is used in the treatment of acute myeloid leukemia; anti-CD20 antibodies including ibritumomab (ZEVALIN™) which is used in the treatment of lymphoma, rituximab (RITUXAN™) which is used in the treatment of Hodgkin lymphoma, tositumomab (BEXXAR™) which is used in the treatment of Hodgkin lymphoma and ofatumumab (ARZERRA™) which is used in the treatment of chronic lymphocytic leukemia; anti-EGFR antibodies such as cetuximab (ERBITUX™) which is used in the treatment of colorectal cancer, head and neck cancer, and squamous cell carcinoma, and panitumumab (VECTIBEX™) which is used in the treatment of colorectal cancer; anti-Her2 antibodies, including trastuzumab (HERCEPTIN™) which is used in the treatment of breast cancer and stomach cancer; anti-CTLA4 antibodies including Ipilimumab (YERVOY™) which is used in the treatment of melanoma.

In certain embodiments, a selective c-FLIP inhibitor of the present invention, or a pharmaceutical composition thereof, is administered in combination with a TRAIL-based anticancer agent in order to treat a cancer, in particular a cancer that is resistant or insensitive to TRAIL receptor ligands or agonists. Indeed, the present Inventors have experimentally shown that treatment of cells from a human lung cancer cell line that overexpresses the c-FLIP protein, with TRAIL alone had no effect on cell death, because of the anti-apoptotic role of c-FLIP. However, the combination of TRAIL with a selective c-FLIP inhibitor of the invention was found to enhance TRAIL-mediated apoptosis, which confirms that a selective c-FLIP inhibitor of the invention efficiently prevents c-FLIP from binding to the death receptor, thus restoring the caspase-8 apoptotic cascade (see Examples section below).

As used herein, the term ***"TRAIL"*** refers to the tumor necrosis factor (TNF)-related apoptosis-inducing ligand, also known as Apo-2 ligand (Apo2L), which is a member of the cytokine superfamily. By cross-linking TRAIL-Receptor 1 (TRAIL-R1) or TRAIL-Receptor 2 (TRAIL-R2), also known as death receptors 4 and 5 (DR4 and DR5, respectively), TRAIL has the capability to induce apoptosis in a wide variety of tumor cells while sparing vital normal cells. The discovery of this unique property led to the development of TRAIL-based anticancer agents.

The terms ***"TRAIL-R1", "DR4"*** and ***"DR4 receptor"*** are used herein interchangeably. They refer to the full length TRAIL receptor sequence and soluble, extracellular domain forms of the receptor described in Pan et al., Science, 1997, 276: 111-113; WO98/32856; U.S. Pat. No. 6,342,363; and WO99/37684.

The terms ***"TRAIL-R2", "DR5"*** and ***"DR5 receptor"*** are used herein interchangeably. They refer to the full length TRAIL receptor sequence and soluble, extracellular domain forms of the receptor described in Sheridan et al., Science, 1997, 277: 818-821; Pan et al., Science, 1997, 277: 815-818; U.S. Pat. No. 6,072,047; U.S. Pat. No. 6,342,369; WO98/51793; WO98/41629; Screaton et al., Curr. Biol., 1997, 7: 693-696; Walczak et al., EMBO J., 1997, 16: 5386-5387; Wu et al., Nature Genetics, 1997, 17: 141-143; WO98/35986; WO98/46643; WO99/02653; WO99/09165; and WO99/11791.

The term ***"TRAIL-based anticancer agents",*** as used herein, refers to any molecule or compound that interacts or interferes with TRAIL normal activity to produce a therapeutic effect. The therapeutic effect may result from any of a large variety of action mechanisms. Examples of TRAIL-based anticancer agents include, in particular, TRAIL receptor ligands and TRAIL receptor agonist.

The term ***"TRAIL receptor ligand",*** as used herein, refers TRAIL, recombinant TRAIL, TRAIL fragments and TRAIL analogues that act as ligands to one of the TRAIL receptors (*i.e.,* that selectively bind to one of the TRAIL receptors to form a complex), in particular to TRAIL-R1 (DR4) and/or TRAIL-R2 (DR5)

The term ***"TRAIL receptor agonist"*** refers to any molecule or compound that partially or fully enhances, stimulates or activates one or more biological activities of TRAIL-R1 or TRAIL-R2, and biologically active variants thereof, whether *in vitro, in situ, in vivo* or *ex vivo* as a result of its direct binding to one or both of these receptors, which causes receptor activation or signal transduction. In the practice of the present invention, the biological activity of TRAIL-R1 or TRAIL-R2 that is partially or fully enhanced, stimulated or activated is apoptosis.

The ligands and agonists of the TRAIL receptors that are useful in the practice of the methods of treatment of the present invention are typically TRAIL peptides or mimics, such as TRAIL fragments, variants or fusions thereof, which may optionally be linked to a conjugate molecule that extends the *in vivo* half-life of the TRAIL conjugate compared to the TRAIL fragments, variants or fusions in the absence of the conjugate molecule. Nucleic acid an amino acid sequences for human TRAIL are known in the art. Preferably, the TRAIL fragment is a soluble TRAIL. Endogenous, full-length TRAIL includes a cytoplasmic domain, a transmembrane domain, and an extracellular domain. Typically, soluble TRAIL is the extracellular domain of TRAIL or a functional fragment thereof or variant thereof that can agonize signalling through TRAIL-R1 or TRAIL-R2. Dulanermin (Apo1L/TRAIL), for example, is a recombinant human TRAIL which targets both TRAIL-R1 and TRAIL-R2 (see WO 2009/140469). Examples of TRAIL conjugates are known in the art (Kim et al., Bioconjugate Chem., 2011, 22: 1631-1637; Chae et al., Molecular Cancer Therapeutics, 2010, 9: 1719-1729). Examples of TRAIL analogues are known in the art, such as for example DR4-selective mutants of wild-type TRAIL (Tur et al., J. Biol. Chem., 2008, 283: 20560-20568), DR5-selective mutants of wild-type TRAIL (van der Sloot et al.,, PNAS USA, 2006, 103: 8634-8639) and monovalent, divalent and trivalent TRAIL-mimicking peptides (Pavet et al., Cancer Research, 2010, 70: 1101-1110). Examples of TRAIL fusion proteins are known in the art (Gieffers et al., Molecular Cancer Therapeutics, 2013, 12: 27357; Wahl et al., Hepatology, 2013, 57: 625-636). A TRAIL agonist may be an agonistic antibody that binds to the TRAIL-R1 (DR4) receptor or to the TRAIL-R2 (DR5) receptor. Agonistic antibodies, that bind to and activate the receptor, mimicking the natural ligand, TRAIL, are also called ***"anti-TRAIL receptor antibodies".*** Agonistic antibodies that bind to TRAIL-R1 (DR4) include mapatumumab (HGS-ETR1), a fully humanized monoclonal antibody. Agonistic antibodies that bind to TRAIL-R2 (DR5) include lexatumumab (HGS-RTR2), drozitumab (Apomab/PRO95780), conatumumab (AMG 655), tigatuzumab (CS-1008/TRA-8), HGSTR2J/KMTRS and LBY-135. Lexatumumab, drozitumab, and conatumumab are fully human IgG1 antibodies, while tigatuzumab is a humanized IgG1 antibody and LBY135 is a chimeric mouse/human antibody. TRAIL receptors and antibodies that bind to TRAIL receptors are described in U.S. Pat. Nos. 6,455,040 (DRS); 6,743,625 (DR5); 6,433,147 (DR4), and 6,902,910 (DR4). Other examples include 7, 115, 717 anti TRAIL-R receptors; 2002097033 anti TRAIL-R4; 20060062786 anti TRAIL-R; 2014161845 anti-DR5 mapatumumab and drozitumab; 2014159562 anti-DR5 lexatumumab and tigatuzumab and LBY 135; 2013148877 conatumumab.

Thus, the present invention provides for the use of at least one of the nine selective c-FLIP inhibitors described herein to improve the sensitivity of a cancer patient to TRAIL receptor agonist therapy. As used herein, the term ***"TRAIL receptor agonist therapy"*** refers to a therapy (or method of treatment) wherein a TRAIL-based anticancer agent (or TRAIL receptor ligand or agonist) is administered to the subject treated. The present invention also provides a method of improving the sensitivity of a cancer patient to TRAIL receptor agonist therapy, said method comprising steps of:
(1) identifying a cancer patient as a candidate for TRAIL receptor agonist therapy or a cancer patient treated using a receptor agonist therapy, and (2) administering to the patient an effective amount of at least one of the nine selective c-FLIP inhibitors of the invention and an effective amount of at least one TRAIL receptor ligand or agonist, wherein the at least one of the nine selective c-FLIP inhibitors is administered before, during or after administration of the at least one TRAIL receptor ligand or agonist.

Thus, in certain embodiments, a selective c-FLIP inhibitor of the present invention, or a pharmaceutical composition thereof, is administered in combination with a TRAIL-based anticancer agent.

In methods where at least one selective c-FLIP inhibitor is administered in combination with at least one additional therapeutic agent, the c-FLIP inhibitor and the additional therapeutic agent may be administered concurrently *(i.e.,* together or separately but at about the same time, *e.g.*, within 5 minutes, 15 minutes or 30 minutes of each other), or alternatively, they may be administered sequentially (*i.e.,* separately and at different times, *e.g*., different times of the same day or different times of the same week or different times of the same month, etc...).

### C. Administration

A selective c-FLIP inhibitor according to the present invention (optionally after formulation with one or more appropriate pharmaceutically acceptable carriers or excipients), in a desired dosage, can be administered to a subject in need thereof by any suitable route. Various delivery systems are known and can be used to administer a selective c-FLIP inhibitor of the present invention, including tablets, capsules, injectable solutions, encapsulation in liposomes, microparticles, microcapsules, etc. Methods of administration include, but are not limited to, dermal, intradermal, intramuscular, intraperitoneal, intralesional, intravenous, subcutaneous, intranasal, pulmonary, epidural, ocular, and oral routes. A selective c-FLIP inhibitor of the present invention, or a pharmaceutical composition thereof, may be administered by any convenient or other appropriate route, for example, by infusion or bolus injection, by adsorption through epithelial or mucocutaneous linings (*e.g*., oral, mucosa, rectal and intestinal mucosa, etc). Administration can be systemic or local. Parenteral administration may be directed to a given tissue of the patient, such as by catheterization. As will be appreciated by those of ordinary skill in the art, in embodiments where a selective c-FLIP inhibitor is administered along with an additional therapeutic agent, the c-FLIP inhibitor and the therapeutic agent may be administered by the same route (*e.g.,* orally) or by different routes (*e.g.,* orally and intravenously).

### D. Dosage

Administration of a selective c-FLIP inhibitor (or a pharmaceutical composition thereof) according to the present invention will be in a dosage such that the amount delivered is effective for the intended purpose. The route of administration, formulation dosage administered will depend upon the therapeutic effect desired, the severity of the disorder being treated, the presence of any infection, the age, sex, weight and general health condition of the patient as well as upon the potency, bioavailability and *in vivo* half-life of the selective c-FLIP inhibitor used, the use (or not) of concomitant therapies, and other clinical factors. These factors are readily determinable by the attending physician in the course of the therapy. Alternatively or additionally, the dosage to be administered can be determined from studies using animal models. Adjusting the dose to be achieve maximal efficacy based on these and other methods are well known in the art and are within the capabilities of trained physicians.

A treatment according to the present invention may consist of a single dose or multiple doses. Thus, administration of a selective c-FLIP inhibitor, or of a pharmaceutical composition thereof, may be constant for a certain period of time or periodic and at specific intervals, *e.g*., hourly, daily, weekly (or at some other multiple day interval); monthly, yearly (*e.g.,* in a time release form). Alternatively, the delivery may occur at multiple times during a given time period, *e.g*., two or more times per week, two or more times per month, and the like. The delivery may be continuous delivery for a period of time, e.g., intravenous delivery.

In general, the amount of the selective c-FLIP inhibitor (or a pharmaceutical composition thereof) administered will preferably be in the range of about 1 ng/kg to about 1000 mg/kg body weight of the subject, for example, between about 100 ng/kg and about 500 mg/kg body weight of the subject; or between about 1 µg/kg and about 100 mg/kg body weight of the subject; or between about 100 µg/kg and about 10 mg/kg body weight of the subject; or between about 500 µg/kg and about 1 mg/kg body weight of the subject.

### III - Pharmaceutical Compositions

As mentioned above, a selective c-FLIP inhibitor of the present invention may be administered *per se* or as a pharmaceutical composition. Accordingly, the present invention provides pharmaceutical compositions comprising an effective amount of a selective c-FLIP inhibitor and at least one pharmaceutically acceptable carrier or excipient for use in the treatment of cancer. In some embodiments, the composition further comprises one or more additional biologically active agents.

A selective c-FLIP inhibitor of the invention, or a pharmaceutical composition thereof, may be administered in any amount and using any route of administration effective for achieving the desired prophylactic or therapeutic effect. The optimal pharmaceutical formulation can be varied depending upon the route of administration and desired dosage. Such formulations may influence the physical state, stability, rate of *in vivo* release, and rate of *in vivo* clearance of the administered active ingredient.

The pharmaceutical compositions of the present invention may be formulated in dosage unit form for ease of administration and uniformity of dosage. The expression "unit dosage form", as used herein, refers to a physically discrete unit for the patient to be treated. It will be understood, however, that the total daily dosage of the compositions will be decided by the attending physician within the scope of sound medical judgement.

### A. Formulation

Injectable preparations, for example, sterile injectable aqueous or oleaginous suspensions may be formulated according to the known art using suitable dispersing or wetting agents, and suspending agents. The sterile injectable preparation may also be a sterile injectable solution, suspension or emulsion in a non-toxic parenterally acceptable diluent or solvent, for example, as a solution in 2,3-butanediol. Among the acceptable vehicles and solvents that may be employed are water, Ringer's solution, U.S.P. and isotonic sodium chloride solution. In addition, sterile, fixed oils are conventionally employed as a solution or suspending medium. For this purpose any bland fixed oil can be employed including synthetic mono- or di-glycerides. Fatty acids such as oleic acid may also be used in the preparation of injectable formulations. Sterile liquid carriers are useful in sterile liquid form compositions for parenteral administration.

Injectable formulations can be sterilized, for example, by filtration through a bacterial-retaining filter, or by incorporating sterilizing agents in the form of sterile solid compositions which can be dissolved or dispersed in sterile water or other sterile injectable medium prior to use. Liquid pharmaceutical compositions which are sterile solutions or suspensions can be administered by, for example, intravenous, intramuscular, intraperitoneal or subcutaneous injection. Injection may be *via* single push or by gradual infusion. Where necessary or desired, the composition may include a local anesthetic to ease pain at the site of injection.

In order to prolong the effect of an active ingredient, it is often desirable to slow the absorption of the ingredient from subcutaneous or intramuscular injection. Delaying absorption of a parenterally administered active ingredient may be accomplished by dissolving or suspending the active ingredient in an oil vehicle. Injectable depot forms are made by forming micro-encapsulated matrices of the active ingredient in biodegradable polymers such as polylactide-polyglycolide. Depending upon the ratio of active ingredient to polymer and the nature of the particular polymer employed, the rate of ingredient release can be controlled. Examples of other biodegradable polymers include poly(orthoesters) and poly(anhydrides). Depot injectable formulations can also be prepared by entrapping the active ingredient in liposomes or microemulsions which are compatible with body tissues.

Liquid dosage forms for oral administration include, but are not limited to, pharmaceutically acceptable emulsions, microemulsions, solutions, suspensions, syrups, elixirs, and pressurized compositions. In addition to the selective c-FLIP inhibitor , the liquid dosage form may contain inert diluents commonly used in the art such as, for example, water or other solvent, solubilising agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cotton seed, ground nut, corn, germ, olive, castor, and sesame oils), glycerol, tetrahydrofurfuryl alcohol, polyethylene glycols, and fatty acid esters of sorbitan and mixtures thereof. Besides inert diluents, the oral compositions can also include adjuvants such as wetting agents, suspending agents, preservatives, sweetening, flavouring, and perfuming agents, thickening agents, colors, viscosity regulators, stabilizes or osmo-regulators. Examples of suitable liquid carriers for oral administration include water (potentially containing additives as above, e.g., cellulose derivatives, such as sodium carboxymethyl cellulose solution), alcohols (including monohydric alcohols and polyhydric alcohols such as glycols) and their derivatives, and oils (*e.g*., fractionated coconut oil and arachis oil). For pressurized compositions, the liquid carrier can be halogenated hydrocarbon or other pharmaceutically acceptable propellant.

Solid dosage forms for oral administration include, for example, capsules, tablets, pills, powders, and granules. In such solid dosage forms, the selective c-FLIP inhibitor may be mixed with at least one inert, pharmaceutically acceptable excipient or carrier such as sodium citrate or dicalcium phosphate and one or more of: (a) fillers or extenders such as starches, lactose, sucrose, glucose, mannital, and silicic acid; (b) binders such as, for example, carboxymethylcellulose, alginates, gelatine, polyvinylpyrrolidone, sucrose, and acacia; (c) humectants such as glycerol; (d) disintegrating agents such as agar-agar, calcium carbonate, potato or tapioca starch, alginic acid, certain silicates, and sodium carbonate; (e) solution retarding agents such as paraffin; absorption accelerators such as quaternary ammonium compounds; (g) wetting agents such as, for example, cetyl alcohol and glycerol monostearate; (h) absorbents such as kaolin and bentonite clay; and (i) lubricants such as talc, calcium stearate, magnesium stearate, solid polyethylene glycols, sodium lauryl sulfate, and mixtures thereof. Other excipients suitable for solid formulations include surface modifying agents such as non-ionic and anionic surface modifying agents. Representative examples of surface modifying agents include, but are not limited to, poloxamer 188, benzalkonium chloride, calcium stearate, cetostearyl alcohol, cetomacrogol emulsifying wax, sorbitan esters, colloidal silicon dioxide, phosphates, sodium dodecylsulfate, magnesium aluminum silicate, and triethanolamine. In the case of capsules, tablets and pills, the dosage form may also comprise buffering agents.

Solid compositions of a similar type may also be employed as fillers in soft and hard-filled gelatine capsules using such excipients as lactose or milk sugar as well as high molecular weight polyethylene glycols and the like. The solid dosage forms of tablets, dragees, capsules, pills, and granules can be prepared with coatings and shells such as enteric coatings, release controlling coatings and other coatings well known in the pharmaceutical formulating art. They may optionally contain opacifying agents and can also be of a composition such that they release the active ingredient(s) only, or preferably, in a certain part of the intestinal tract, optionally, in a delaying manner. Examples of embedding compositions which can be used include polymeric substances and waxes.

In certain embodiments, it may be desirable to administer an inventive composition locally to a specific area. This may be achieved, for example, and not by way of limitation, by local infusion during surgery, topical application, by injection, by means of a catheter, by means of suppository, or by means of a skin patch or stent or other implant.

For topical administration, the composition is preferably formulated as a gel, an ointment, a lotion, or a cream which can include carriers such as water, glycerol, alcohol, propylene glycol, fatty alcohols, triglycerides, fatty acid esters, or mineral oil. Other topical carriers include liquid petroleum, isopropyl palmitate, polyethylene glycol, ethanol (95%), polyoxyethylenemonolaurat (5%) in water, or sodium lauryl sulfate (5%) in water. Other materials such as antioxidants, humectants, viscosity stabilizers, and similar agents may be added as necessary.

In addition, in certain instances, it is expected that the inventive compositions may be disposed within transdermal devices placed upon, in, or under the skin. Such devices include patches, implants, and injections which release the active ingredient by either passive or active release mechanisms. Transdermal administrations include all administrations across the surface of the body and the inner linings of bodily passage including epithelial and mucosal tissues. Such administrations may be carried out using the present compositions in lotions, creams, foams, patches, suspensions, solutions, and suppositories (rectal and vaginal).

Transdermal administration may be accomplished through the use of a transdermal patch containing an active ingredient (*i.e.,* a selective c-FLIP inhibitor according to the present invention) and a carrier that is non-toxic to the skin, and allows the delivery of the ingredient for systemic absorption into the bloodstream *via* the skin. The carrier may take any number of forms such as creams and ointments, pastes, gels, and occlusive devices. The creams and ointments may be viscous liquid or semisolid emulsions of either the oil-in-water or water-in-oil type. Pastes comprised of absorptive powders dispersed in petroleum or hydrophilic petroleum containing the active ingredient may be suitable. A variety of occlusive devices may be used to release the active ingredient into the bloodstream such as a semi-permeable membrane covering a reservoir containing the active ingredient with or without a carrier, or a matrix containing the active ingredient.

Suppository formulations may be made from traditional materials, including cocoa butter, with or without the addition of waxes to alter the suppository's melting point, and glycerine. Water soluble suppository bases, such as polyethylene glycols of various molecular weights, may also be used.

Materials and methods for producing various formulations are known in the art and may be adapted for practicing the subject invention. Suitable formulations for the delivery of antibodies can be found, for example, in *"*Remington's Pharmaceutical Sciences", E.W. Martin, 18th Ed., 1990, Mack Publishing Co.: Easton, PA.

### B. Additional Biologically Active Agents

In certain embodiments, a selective c-FLIP inhibitor of the invention is the only active ingredient in a pharmaceutical composition of the present invention. In other embodiments, the pharmaceutical composition further comprises one or more additional biologically active agents. Examples of suitable biologically active agents include, but are not limited to, anti-cancer agents, anti-inflammatory agents, immunomodulatory agents, analgesics, antimicrobial agents, antibacterial agents, antibiotics, antioxidants, antiseptic agents, and combinations thereof. Examples of selective anti-cancer agents, including TRAIL-based therapeutic agents have been listed above.

In such pharmaceutical compositions, the selective c-FLIP inhibitor and the at least one additional therapeutic agent may be combined in one or more preparations for simultaneous, separate or sequential administration of the selective c-FLIP inhibitor and therapeutic agent(s). More specifically, an inventive composition may be formulated in such a way that the selective c-FLIP inhibitor and therapeutic agent(s) can be administered together or independently from each other. For example, the selective c-FLIP inhibitor and a therapeutic agent can be formulated together in a single composition. Alternatively, they may be maintained (*e.g.*, in different compositions and/or containers, for example under the form of a pharmaceutical pack or kit) and administered separately.

### C. Pharmaceutical Packs or Kits

In another aspect, the present invention provides a pharmaceutical pack or kit comprising one or more containers (e.g., vials, ampoules, test tubes, flasks or bottles) containing one or more ingredients allowing administration of a selective c-FLIP inhibitor of the present invention.

Different ingredients of a pharmaceutical pack or kit may be supplied in a solid (*e.g*., lyophilized) or liquid form. Each ingredient will generally be suitable as aliquoted in its respective container or provided in a concentrated form. Packs or kits according to the invention may include media for the reconstitution of lyophilized ingredients. Individual containers of the kits will preferably be maintained in close confinement for commercial sale.

In certain embodiments, a pack or kit includes one or more additional therapeutic agent(s), such as those listed above. Optionally associated with the container(s) can be a notice or package insert in the form prescribed by a governmental agency regulating the manufacture, use or sale of pharmaceutical or biological products, which notice reflects approval by the agency of manufacture, use or sale for human administration. The notice of package insert may contain instructions for use of a pharmaceutical composition according to methods of treatment disclosed herein.

An identifier, *e.g.,* a bar code, radio frequency, ID tags, etc., may be present in or on the kit. The identifier can be used, for example, to uniquely identify the kit for purposes of quality control, inventory control, tracking movement between workstations, etc.

### Examples

The following examples describe some of the preferred modes of making and practicing the present invention. However, it should be understood that the examples are for illustrative purposes only and are not meant to limit the scope of the invention. Furthermore, unless the description in an Example is presented in the past tense, the text, like the rest of the specification, is not intended to suggest that experiments were actually performed or data were actually obtained.

### Materials and Methods

***Cell Culture***. H1703 (Human non-small lung cancer cell line) cells were grown in RPMI 1640 (LONZA) culture media supplemented with 10 % fetal bovine serum and puromycine antibiotic (2 µg/ml) from Sigma-Aldrich. The cells were kept in a humidified atmosphere in an incubator at 37°C and 5 % CO₂. H1703 were a kind gift from O. Micheau (INSERM, Dijon, France).

***Flow Cytometry Analysis***. The apoptotic cell death was confirmed by flow cytometry (cytoFLEX, Backman Coulter). After treatment of cells with TRAIL alone, c-FLIP inhibitor alone, or co-admninistration TRAIL/c-FLIP inhibitor, the cells were collected, washed and re-suspended in 1X Annexin binding buffer. Annexin-V- PE was added to cells, and left 20 minutes at room temperature in the dark. Flow cytometric analysis is done in the final step.

***Transformation of Competent Bacteria***. FLIP (s) and FADD genes were previously cloned and inserted in their respective plasmids pET24b and pMALC2, and provided as a kind gift from Pr. Richard Daniellou (ICOA, Orléans, France). Pet24b and Pmalc2 plasmids were mixed with their corresponding competent bacterial cells, Rosetta and DH5α cells respectively, and left on ice for 20 minutes. Heat-shocking steps were performed before adding LB medium and leaving cells to grow on 37°C for 1 hour and then cells were spread on LB plates with appropriate antibiotics and incubated at 37°C overnight.

***Production of c-FLIP (s) and FADD proteins.*** Transformed competent cells were cultured in large amounts of LB growth at 37°C and the expression of each protein was stimulated using 1M IPTG (Isopropyl β-D-1-thiogalactopyranoside) and cells were left to grow for 18 hours at 30°C. Cells were collected, centrifuged and resuspended in Tris/NaCl buffer (Tris 50 mM, NaCl 100 mM, pH 8.0) after which they were centrifuged at 6000 g, for 10 minutes at 4°C. Supernatant was removed and bacterial pellets were used for cell lysis and protein purification on Ni-NTA column and MBP-trap column for cFLIP(s) and FADD purification, respectively. The FLIP(s) is a His-tagged protein, and FADD is a MBP-tagged protein. The purified proteins were incubated with each molecule for 18 hours +4°C and then, the samples were passed again on MBP-trap columns.

***Western Blot Analysis.*** The samples purified and obtained after FADD/FLIP/compounds incubation were run on 4-12 % Bis-Tris polyacrylamide gels to evaluate the presence of FLIP and FADD bands.

***Reagents and Antibodies.*** KillerTRAIL (human recombinant BULK) was purchased from Alexis Biochemicals. The 9 molecules were obtained from NCI -DS & CB (National cancer institute- Drug synthesis and chemistry branch, USA). For Western blotting experiments, anti-FLIP antibody (clone DAVE 2) was purchased from ADIPOGEN. Anti-His antibody (3D5) was purchased from Invitrogen. Anti-MBP antibody was purchased from New England Biolabs. Anti-rabbit and anti-mouse HRP linked secondary antibodies were purchased from Santa Cruz Biotechnology. Annexin V-PE apoptosis detection kit was purchased from BD Biosciences and used according to the manufacturer's instructions.

### Results

**Identification of Selective c-FLIP Inhibitors.** Since the crystallographic structures of caspase-8 and c-FLIP are unknown, the present Inventors have resorted to the construction of c-FLIP and of caspace-8 with "3D homology models" using *in silico* methods (MOE2012, SiteFinder, GeneDoc). They found that the Death Effector Domain 2 (DED 2) of c-FLIP is homologous to the DED 2 of viral v-FLIP and the DED 2 of caspase-8 is homologous to the DED of FADD.

Docking methods were then used to identify, among a library of 1880 molecules available from the NIH, the molecules which were able to selectively bind to c-FLIP *versus* caspase-8. Nine molecules having chemical formula **(1)-(9)** were identified by this process.

**Assessment of the *in vitro* Properties of the 9 Molecules Identified.** To assess the properties of the nine molecules identified, the present Inventors used cells from the human lung cancer cell line H1703 that overexpresses c-FLIP.

***Sensitivity of the H1703 cells to TRAIL:*** The first goal was to verify that the H1703 cells are sensitive to TRAIL. To do so, the cells were treated with TRAIL at a concentration of 100 ng/mL for 18 hours and then the cells were washed and incubated with Annexin V-PE to evaluate the apoptosis level using flow cytometry. As expected, the H1703-Mock cells (that are lacking the c-FLIP(L), were sensitive to TRAIL and apoptosis reached a level of more than 60%. In contrast, H1703-FLIP-L) cells were found to be resistant to TRAIL, due to the expression of the anti-apoptotic FLIP protein. The results obtained, which are presented in Figure 1(A), clearly show that the cells of the H1703 cell line are TRAIL-sensitive. H1703 cells were then threated with each molecule alone at a standard concentration of 25 µM to evaluate the molecule cytotoxicity (see Figure 1(B)). Some molecules were found to be cytotoxic at 25 µM while other exhibited no cytotoxicity. So, the Inventors applied a range of concentrations for each molecule to determine the appropriate dose that has no toxic effect on the cells when administered alone.

***Combination Treatment of TRAIL with each one of the 9 Identified Molecules:*** Following the identification of the best concentration for each compound, HE1703 cells were treated with TRAIL (100 ng/mL) alone or with one of the 9 identified molecules alone or with a combination of TRAIL and one of the 9 identified molecules for 18 hours. The percentage of apoptosis was measured by flow cytometry using an annexin V test, as described above. The results obtained are presented on Figure 2(A, B and C). They show that the death ligand TRAIL had no effect on cells overexpressing the anti-apoptotic protein c-FLIP, while blocking c-FLIP function with any one of the 9 identified molecules increased the sensitivity of tumor cells to TRAIL-induced apoptosis.

***Pull Down Assay:*** The ability of the nine identified molecules to inhibit the interaction between FADD and c-FLIP was then checked using a pull down assay. After the evaluation of the inhibitory role of the nine c-FLIP inhibitors using an in vitro test, the present Inventors wanted to confirm the results using a biochemical and molecular test to demonstrate that these molecules can indeed inhibit the interaction between FADD and c-FLIP. To this end, they produced c-FLIP(s) (because c-FLIP(s) does not need purification due to its highly efficient purification) and FADD in bacteria, and then they purified these proteins using appropriate columns (as described above). Once purified, c-FLIP(s) and FADD were incubated for 18 hours with each one of the nine c-FLIP inhibitors at different concentrations. The samples were then passed on a MBP-trap column, which binds FADD (MBP-tagged protein). If cFLIP is still bound to FADD, then it is captured with FADD on the MBP column. Samples were then evaluated using a Western blot analysis by monitoring the disappearance of the c-FLIP band with increasing concentration of the c-FLIP inhibitor. The results obtained are presented in Figure 3(A, B and C). Disappearance of the c-FLIP band in Western Blot corresponds to FADD-c-FLIP interaction inhibition. The results obtained show that the c-FLIP inhibitors with formulas **(1), (2), (3), (4), (7)** and **(9)** inhibit the interaction between FADD and c-FLIP, while the molecules with formulas **(6),** and **(8)** were found to have no effect as the band corresponding to c-FLIP was always present even at high concentrations. These *in vitro* and molecular experimental results confirm that the majority of the nine molecules of the present invention are indeed c-FLIP inhibitors.

## Claims

1. A selective c-FLIP inhibitor, or a physiologically acceptable salt thererof, for use in the treatment of cancer, wherein said selective c-FLIP inhibitor is a small molecule having one of following formulas: 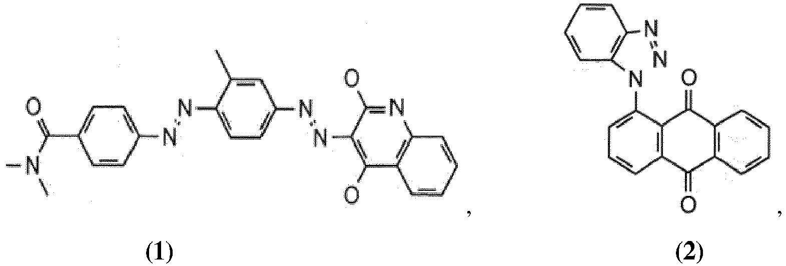 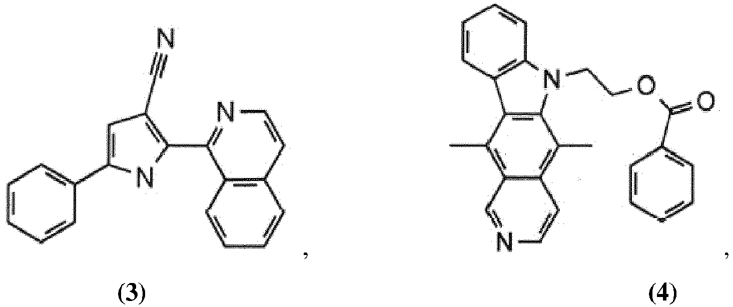 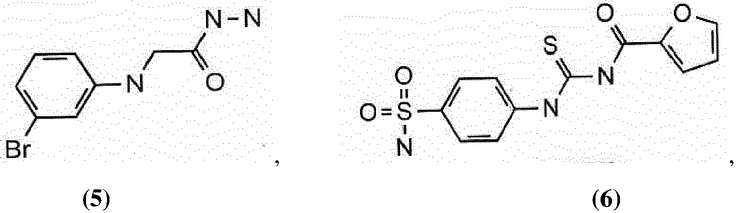 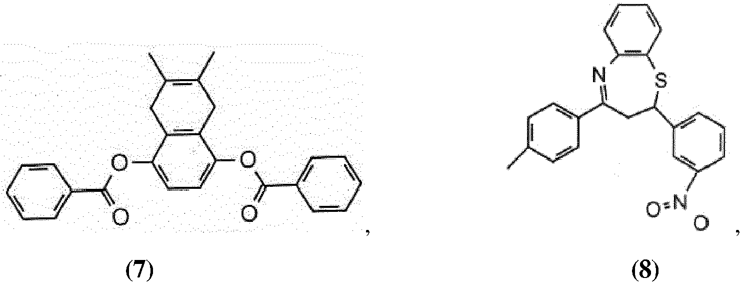 and

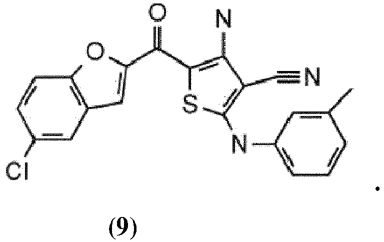

2. The selective c-FLIP inhibitor, or physiologically acceptable salt thereof, for the use according to claim 1, wherein said selective c-FLIP inhibitor is a small molecule having one of formulas **(1), (4),** and **(9).**

3. The selective c-FLIP inhibitor, or physiologically acceptable salt thereof, for the use according to claim 1 or claim 2, wherein the cancer overexpresses c-FLIP.

4. The selective c-FLIP inhibitor, or physiologically acceptable salt thereof, for the use according to claim 3, wherein the cancer that overexpresses c-FLIP is selected from the group consisting of lung cancer, colorectal cancer, pancreatic cancer, ovarian cancer, gastric cancer, breast cancer, prostate cancer, melanoma, gliobastoma, bladder urothelial cancer, cervical cancer, Burkitt's lymphoma, non-Hodgkin's lymphoma, head and neck squamous cell carcinoma (HNSCC), hepatocellular carcinomas, B cell chronic lymphocytic leukemia, gallbladder carcinoma, Ewing sarcoma, nasopharyngeal carcinoma, follicular lymphoma, Acute Lymphoblastic Leukemia (ALL), neuroblastoma, myeloma, acute myeloid leukemia, osteosarcoma, renal sarcoma, endometrial carcinoma, malignant pleural mesothelioma, Kaposi's sarcoma, peripheral T-cell lymphoma, and erythroleukemia.

5. The selective c-FLIP inhibitor, or physiologically acceptable salt thereof, for the use according to any of claims 1 to 4, wherein the cancer is chemoresistant.

6. The selective c-FLIP inhibitor, or physiologically acceptable salt thereof, for the use according to any of claims 1 to 4, wherein the cancer is resistant or insensitive to a TRAIL-based anticancer agent.

7. The selective c-FLIP inhibitor, or physiologically acceptable salt thereof, for the use according to claim 6, wherein the TRAIL-based anticancer agent is a TRAIL receptor agonist or ligand, in particular a TRAIL-R1 agonist or ligand or a TRAIL-R2 agonist or ligand.

8. A selective c-FLIP inhibitor having one of formulas **(1)-(9),** or a physiologically acceptable salt thereof, for use as a pro-apoptotic agent in the treatment of cancer.

9. A selective c-FLIP inhibitor having one of formulas **(1)-(9),** or a physiologically acceptable salt thereof, for use to overcome the chemoresistance of a cancer.

10. A selective c-FLIP inhibitor having one of formulas **(1)-(9),** or a physiologically acceptable salt thereof, for use to overcome the resistance of a cancer to a TRAIL-receptor agonist therapy or to improve the sensitivity of a cancer to a TRAIL-receptor agonist therapy.

11. A pharmaceutical composition for use in the treatment of cancer or for use to overcome the chemoresistance of a cancer or for use to overcome the resistance of a cancer to a TRAIL-receptor agonist therapy or to improve the sensitivity of a cancer to a TRAIL-receptor agonist therapy, wherein said pharmaceutical composition comprises an effective amount of at least one selective c-FLIP inhibitor having one of formulas **(1)-(9)** and a pharmaceutically acceptable carrier or excipient thereof.

12. The pharmaceutical composition for the use according to claim 11, further comprising at least one additional therapeutic agent.

13. The pharmaceutical composition for the use according to claim 12, wherein the additional therapeutic agent is selected from the group consisting of anti-cancer agents, anti-inflammatory agents, immunomodulatory agents, analgesics, antimicrobial agents, antibacterial agents, antibiotics, antioxidants, antiseptic agents, and combinations thereof.

14. The pharmaceutical composition for the use according to claim 8, wherein the additional therapeutic agent is a TRAIL-based anticancer agent, preferably a TRAIL receptor agonist or ligand, more preferably a TRAIL-R1 agonist or ligand or a TRAIL-R2 agonist or ligand.

15. A kit for use in the treatment of cancer or for use to overcome the chemoresistance of a cancer or for use to overcome the resistance of a cancer to a TRAIL-receptor agonist therapy or to improve the sensitivity of a cancer to a TRAIL-receptor agonist therapy, wherein said kit comprises at least one selective c-FLIP inhibitor having one of formulas **(1)-(9),** or a pharmaceutical composition thereof, and at least one additional therapeutic agent, wherein the at least one selective c-FLIP inhibitor and at least one additional therapeutic agent are comprised in separate containers.
